# FASCICULE DE BREVET EUROPEEN

(11) **EP 1 185 526 B1**
(45) Date de publication et mention de la délivrance du brevet: **15.09.2004**
(21) Numéro de dépôt: 00945979.3
(22) Date de dépôt: 22.06.2000
(51) Int. Cl.: C07D 409/06, C07D 417/12, C07D 279/02, C07C 237/20, C07H 15/26, C07F 13/00, A61K 49/00, A61P 19/00

(54) **DERIVES D' AMONIUM QUATERNAIRE, LEUR PROCEDE DE PREPARATION ET LEUR USAGE EN PHARMACIE**
QUATERNÄRE AMMONIUM VERBINDUNGEN, VERFAHREN ZU DEREN HERSTELLUNG UND DEREN PHARMAZEUTISCHE VERWENDUNG
NOVEL QUATERNARY AMMONIUM DERIVATIVES, METHOD FOR PREPARING SAME AND PHARMACEUTICAL USE

(30) Priorité: 23.06.1999 FR 9908020
(43) Date de publication de la demande: 13.03.2002
(73) Titulaire: Institut National de la Santé et de la Recherche Médicale, 75654 Paris Cedex 13 (FR)
(72) Inventeur: MADELMONT, Jean-Claude, F-63540 Romagnat (FR); GIRAUD, Isabelle, F-63000 Clermont-Ferrand (FR); NICOLAS, Colette, F-63200 Le Cheix-sur-Morge (FR); MAURIZIS, Jean-Claude, F-63170 Pérignat-les-Sarliève (FR); RAPP, Maryse, F-63960 Veyre-Monton (FR); OLLIER, Monique, F-63540 Romagnat (FR); RENARD, Pierre, F-78150 Le Chesnay (FR); CAIGNARD, Daniel-Henri, F-78230 Le Pecq (FR)
(74) Mandataire: Richebourg, Michel François
(86) Numéro de dépôt international: PCT/FR2000/001731
(87) Numéro de publication internationale: WO 2001/000621

(56) Documents cités:
- EP-A- 0 208 404
- WO-A-97/08146

## Description

La présente invention concerne de nouveaux dérivés d'ammonium quaternaire, leur procédé de préparation et les compositions pharmaceutiques qui les contiennent.

Ces nouveaux dérivés d'ammonium quaternaire permettent la vectorisation dans les tissus cartilagineux de principes actifs et ainsi le traitement des pathologies dues à une atteinte du cartilage qu'il s'agisse de pathologies articulaires ou cancéreuses. Ils peuvent être également utilisés à titre de réactifs de diagnostic, capables notamment de révéler une pathologie du cartilage ou un métabolisme (marqueur radioactif, marqueur coloré, ...).

Les agents thérapeutiques actuellement commercialisés pour le traitement des pathologies articulaires comme l'arthrite ou l'arthrose présentent en général une faible affinité pour les tissus cibles et nécessitent des administrations à doses élevées pour obtenir l'effet thérapeutique souhaité.

Ces administrations à forte dose de principes actifs ont pour conséquence une augmentation de la fréquence des effets secondaires. Il est notamment connu que la prise d'anti-inflammatoires non stéroïdiens provoque une importante toxicité digestive.

Dans le domaine de la cancérologie osseuse, il est également connu que les agents thérapeutiques actuellement utilisés pour le traitement des chondrosarcomes notamment présentent des effets secondaires indésirables, en particulier des toxicités notamment hématologiques ou non.

Enfin, dans le domaine des produits de diagnostics des pathologies cartilagineuses, les produits utilisés actuellement présentent l'inconvénient de manquer de spécificité pour les cibles visées.

Il était donc particulièrement intéressant de fonctionnaliser ces différents types de composés de façon à atteindre de manière ciblée le tissu cartilageux et ainsi de limiter, voire de supprimer, les effets indésirables observés lorsque ces composés sont administrés directement.

Les nouveaux composés, objets de la présente invention, permettent, à la fois par l'augmentation du tropisme et par la diminution des doses administrées, d'atténuer significativement les effets secondaires et de renforcer l'index thérapeutique des molécules actives.

Plus spécifiquement, la présente invention concerne les composés de formule répondant à l'une des formules (**Ia**) ou (**Ib**) : 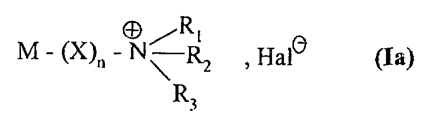 dans laquelle :
- M: représente une molécule utilisable pour la thérapie ou le diagnostic des pathologies dues à une atteinte du cartilage,
- R₁, R₂, R₃,: identiques ou différents, représentent un groupement alkyle (C₁-C₆) linéaire ou ramifié,
ou R₁, R₂, R₃ pris ensemble avec l'atome d'azote qui les porte forment un hétérocycle azoté saturé ou insaturé,
- X: représente une chaîne alkylène (C₁-C₆) linéaire ou ramifiée dans laquelle un ou plusieurs groupements -CH₂- sont éventuellement remplacés par un atome de soufre, d'oxygène, un groupement -NR- (dans lequel R représente un groupement alkyle (C₁-C₆) linéaire ou ramifié), un groupement -CO-, un groupement -CO-NH-, un groupement -CO₂-, un groupement -SO- ou un groupement -SO₂-,
- n: représente 0 ou 1,
- Hal: représente un atome d'halogène,
ou, 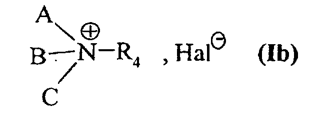
- R₄: représente un groupement alkyle (C₁-C₆) linéaire ou ramifié,
- Hal: représente un atome d'halogène, 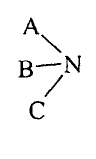
représente une molécule utilisable pour la thérapie ou le diagnostic des pathologies dues à une atteinte du cartilage, étant entendu que l'atome d'azote peut être éventuellement inclus dans un système hétérocyclique azoté saturé ou insaturé, ou engagé dans une double liaison.

Préférentiellement, les composés de formule (Ia) sont les composés tels que :
- n: est égal à 1,
- X: représente une chaîne alkylène (C₁-C₆) linéaire ou ramifiée, un groupement -NR-(CH₂)ₘ- (dans lequel R est tel que défini précédemment), un groupement - CO-(CH₂)ₘ-, ou un groupement -CO-NH-(CH₂)ₘ, dans lesquels m représente un nombre entier compris entre 1 et 5 inclus.

R₁, R₂, R₃ dans les composés de formule **(Ia)** sont préférentiellement les groupements, identiques ou différents, alkyles (C₁-C₆) linéaires ou ramifiés, ou bien sont tels qu'ils forment avec l'atome d'azote qui les porte un cycle pyridine ou pipéridine (et dans ce cas, l'un de ces groupements représente un groupement alkyle (C₁-C₆) linéaire ou ramifié).

Les molécules M ou 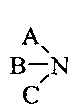 utilisables pour la thérapie ou le diagnostic des pathologies ou le diagnostic des pathologies dues à une atteinte du cartilage sont plus particulièrement : les antiinflammatoires, les antiarthritiques, les antiarthrosiques, les analgésiques, ou les antitumoraux spécifiques.

Les composés préférés de formule **(Ia)** utilisés à titre de principe actif sont :
× les molécules dérivées du *Tenidap* de formule (**Ia**_{**1**}) : 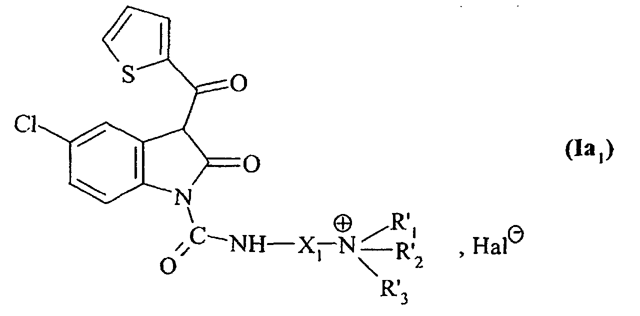 dans laquelle :
   - X₁: représente un groupement alkylène (C₁-C₆) linéaire ou ramifié,
   - R'₁, R'₂, R'₃,: identiques ou différents, représentent un groupement alkyle (C₁-C₆) linéaire ou ramifié,
   - Hal: représente un atome d'halogène,
× les molécules dérivées du *Melphalan* de formule (**Ia**_{**2**}) : 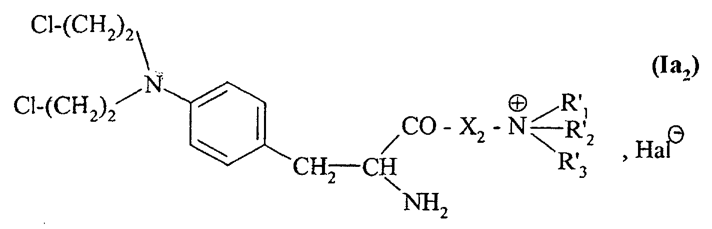 dans laquelle :
   - X₂: représente un groupement -NH-(CH₂)ₘ- dans lequel m est tel que défini précédemment,
   - R'₁, R'₂, R'₃,: sont tels que définis précédemment,
   - Hal: représente un atome d'halogène,
× les molécules dérivées du *Chlorambucil* de formule (**Ia**_{**3**}) : 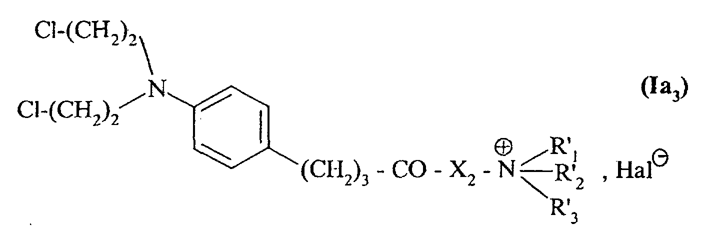 dans laquelle :
   X₂, R'₁, R'₂, R'₃ sont tels que définis précédemment,
   Hal représente un atome d'halogène,
× les molécules dérivées de la *Glucosamine* de formule (**Ia**_{**4**}) : 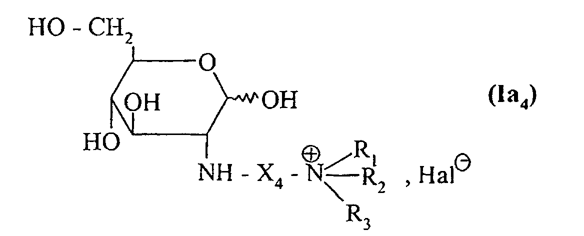 dans laquelle :
   - X₄: représente un groupement -CO-(CH₂)ₘ- dans lequel m est tel que défini précédemment,
   - R₁, R₂, R₃: sont tels que définis précédemment.
   - Hal: représente un atome d'halogène,

Les composés préférés de formule (Ib) utilisés à titre de principe actif sont :
× les molécules dérivées du *Piroxicam* de formule (**Ib**_{**1**}) : 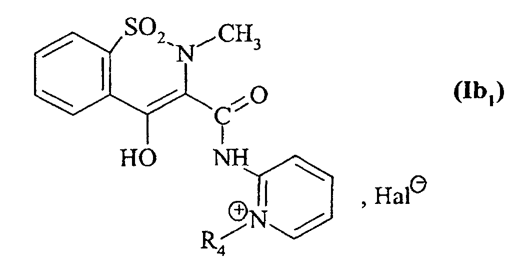 dans laquelle R₄ et Hal sont tels que définis précédemment,
× les molécules de formule (**Ib**_{**2**}) : 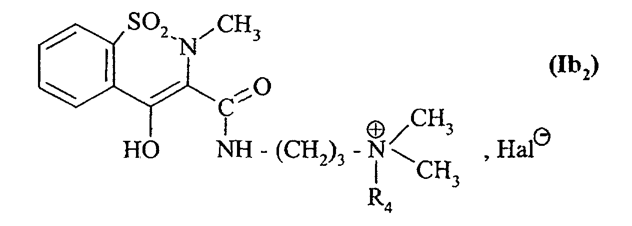 dans laquelle R₄ et Hal sont tels que définis précédemment.

Les composés préférés de formule (Ia) utilisés à titre de réactifs de diagnostic sont les dérivés de formule (**Ia**_{**5**}) : 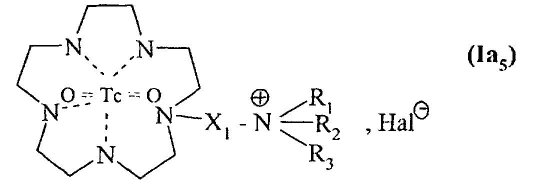 dans laquelle X₁, R₁, R₂, R₃ et Hal sont tels que définis précédemment.

L'invention concerne également le procédé de préparation des composés de formule (Ia) ou (Ib).

Les composés de formule (Ia) sont obtenus selon des procédés classiques de la chimie organique par fonctionnalisation en une ou plusieurs étapes, selon la nature du groupement X souhaité, d'un composé de formule M - P (dans laquelle M a la même signification que dans la formule (Ia) et P représente un atome d'hydrogène ou un groupement hydroxy) ou d'un précurseur du composé de formule M - P suivie des réactions nécessaires à la formation du composé final de formule (Ia).

Les composés de formule (Ib) sont, quant à eux, obtenus par réaction d'un halogénure d'alkyle sur un composé de formule 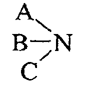 tel que défini précédemment.

Les molécules dérivées du Tenidap de formule (Ia₁) définie précédemment sont obtenues à partir du 5-chloro-2,3-dihydro-2-oxo-1*H*-indole-1-carboxylate de 4-nitrophényle que l'on fait réagir avec une amine de formule (**II**) : 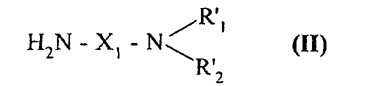 dans laquelle X₁, R'₁ et R'₂ sont tels que définis précédemment, pour conduire au composé de formule (**III**) : 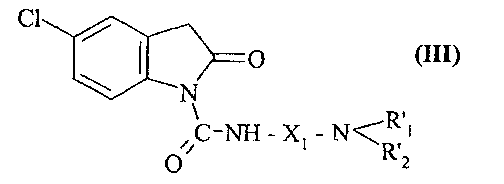 dans laquelle X₁, R'₁ et R'₂ sont tels que définis précédemment,
que l'on soumet à l'action du chlorure de 2-thénoyle en milieu basique sous atmosphère inerte, puis à un traitement acide,
pour conduire au composé de formule (**IV**) : 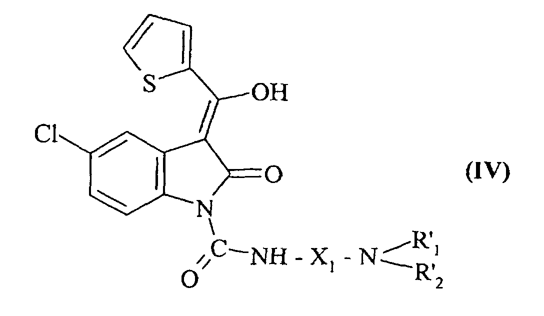 qui est transformé en sel de sodium correspondant,
puis qui subit l'action d'un halogénure d'alkyle (C₁-C₆) linéaire ou ramifié (R'₃Hal), pour conduire au composé de formule (**V**) : 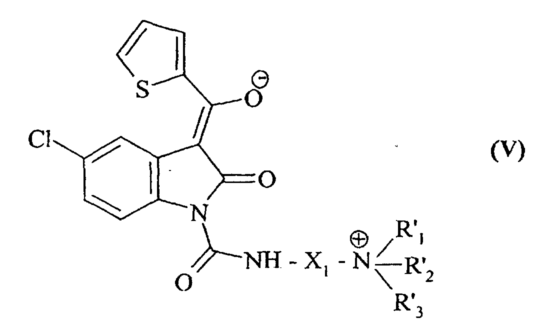 qui, en milieu chlorhydrique, conduit au composé de formule (Ia₁), que l'on purifie le cas échéant.

Les molécules dérivées du Melphalan de formule (Ia₂) définie précédemment sont obtenues à partir du Melphalan dont la fonction amine a été préalablement protégée par un groupement *tert*-butoxycarbonyle (Boc) avec une amine de formule (**VI**) en présence d'un réactif de couplage peptidique : 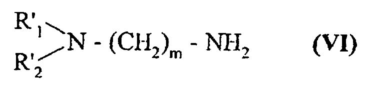 dans laquelle R'₁, R'₂ et m sont tels que définis précédemment,
pour conduire au composé de formule (**VII**) : 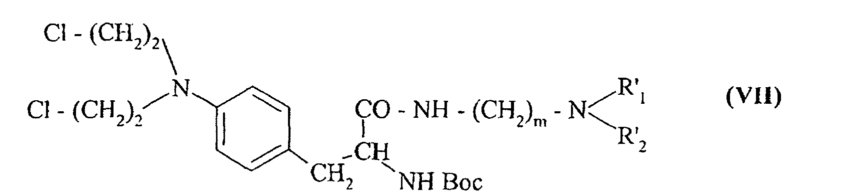 dans laquelle m, R'₁ et R'₂ sont tels que définis précédemment,
que l'on soumet à l'action d'un halogénure d'alkyle (C₁-C₆) linéaire ou ramifié, puis à un traitement chlorhydrique,
pour conduire au composé de formule (Ia₂) que l'on purifie le cas échéant.

Les molécules dérivées du Chlorambucil de formule (Ia₃) définie précédemment sont obtenues à partir du Chlorambucil dont on transforme la fonction acide en chlorure d'acide correspondant,
puis que l'on fait réagir avec une amine de formule (**VI**), en présence ou non d'un réactif de couplage peptidique : 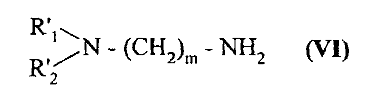 dans laquelle R'₁, R'₂ et m sont tels que définis précédemment,
pour conduire au composé de formule (**VIII**) : 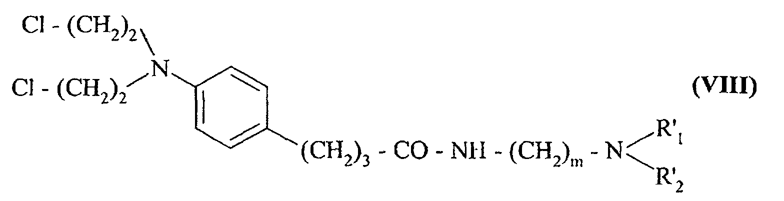 dans laquelle m, R'₁ et R'₂ sont tels que définis précédemment,
que l'on soumet à l'action d'un halogénure d'alkyle (C₁-C₆) linéaire ou ramifié,
pour conduire au composé de formule (Ia₂) que l'on purifie le cas échéant.

Les molécules dérivées de la Glucosamine de formule (Ia₄) définie précédemment sont obtenues par réaction de la Glucosamine avec un chlorure d'acide de formule (**IX**) :

Cl - (CH₂)ₘ - CO - Cl (**IX**)

pour conduire au composé de formule (**X**) : 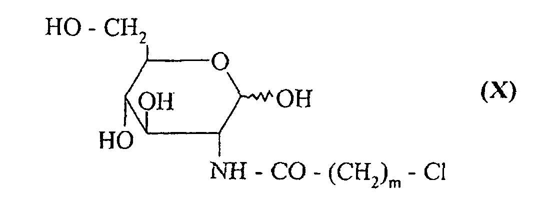 dans laquelle m est tel que défini précédemment,
que l'on condense sur une amine de formule (**XI**) : 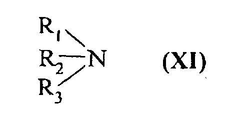 dans laquelle R₁, R₂, R₃ sont tels que définis précédemment,
pour conduire au composé de formule (Ia₄) que l'on purifie le cas échéant et dont on sépare éventuellement les isomères selon une technique classique de séparation.

Les molécules de formule (Ia₅) définie précédemment sont obtenues à partir du composé de formule (**XII**) : 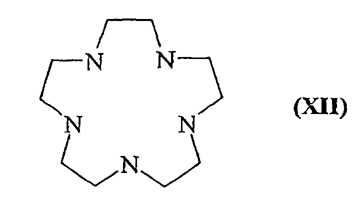 sur lequel on fait réagir un halogénure d'halogénoalkylammonium de formule (**XIII**) : 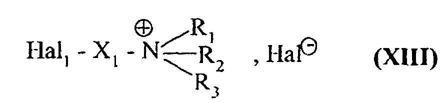 dans laquelle X₁, R₁, R₂, R₃ sont tels que définis précédemment, Hal et Hal₁, identiques ou différents, représentent des atomes d'halogène,
pour conduire au composé de formule (**XIV**) : 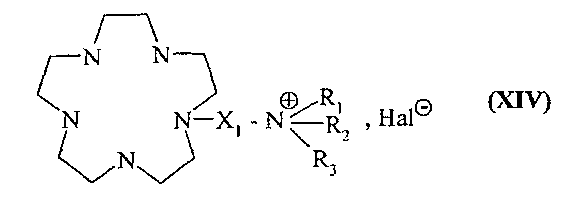 dans laquelle X₁, R₁, R₂, R₃ et Hal sont tels que définis précédemment,
que l'on fait réagir avec du pertechnéate de sodium en présence de chlorure d'étain,
pour conduire au composé de formule (Ia₅) que l'on purifie le cas échéant.

Les molécules dérivées du Piroxicam de formule (Ib₁) définie précédemment sont obtenues à partir du Piroxicam sur lequel on fait réagir un halogénure d'alkyle (C₁-C₆) linéaire ou ramifié, que l'on purifie le cas échéant.

Les molécules de formule (Ib₂) définie précédemment sont obtenues à partir de l'amine correspondante sur laquelle on fait réagir un halogénure d'alkyle (C₁-C₆) linéaire ou ramifié, que l'on purifie le cas échéant.

Les dérivés de la présente invention ont démontré lors d'études biologiques un tropisme renforcé pour les tissus cartilagineux. Ces molécules fonctionnalisées par la fonction ammonium quaternaire ont, d'autre part, des comportements pharmaceutiques très différents de celui des molécules non fonctionnalisées.

Il a été notamment observé une concentration plus élevée dans les cartilages jusqu'à 1 heure après administration.

L'invention s'étend aussi aux compositions pharmaceutiques renfermant comme principe actif au moins un composé de formule (1) avec un ou plusieurs excipients inertes, non toxiques et appropriés. Parmi les compositions pharmaceutiques selon l'invention, on pourra citer plus particulièrement celles qui conviennent pour l'administration orale, parentérale (intraveineuse ou sous-cutanée), nasale, les comprimés simples ou dragéifiés, les comprimés sublinguaux, les gélules, les tablettes, les suppositoires, les crèmes, les pommades, les gels dermiques, les préparations injectables, les suspensions buvables, etc...

La posologie utile est adaptable selon la nature et la sévérité de l'affection, la voie d'administration ainsi que selon l'âge et le poids du patient. Cette posologie varie également selon la nature du composé utilisé.

Les exemples suivants illustrent l'invention mais ne la limitent en aucune façon.

Les produits de départ utilisés sont des produits connus ou préparés selon des modes opératoires connus.

Les structures des composés décrits dans les exemples ont été déterminées selon les techniques spectroscopiques usuelles (infrarouge, RMN, spectrométrie de masse...).

### EXEMPLE 1 : Chlorure de {3-{[(Z)-5-chloro-2,3-dihydro-3-(hydroxy-2-thiénylméthylène)-2-oxo-1H-indol-1-yle]carbonylamino}propyl} triméthylammonium

### STADE A : N-[3-(Diméthylamino)propyl]-5-chloro-2,3-dihydro-2-oxo-1H-indole-1-carboxamide

A une solution contenant 12,08 mmoles de 5-chloro-2,3-dihydro-2-oxo-1*H*-indole-1-carboxylate de 4-nitrophényle dans 70 ml de dichlorométhane, est ajoutée à température ambiante, 12,08 mmoles de 3-(diméthylamino)propylamine. La réaction est immédiate. Après extraction de cette solution par une solution de soude 0,05N jusqu'à ce que la phase aqueuse ne présente plus de coloration jaune, la phase organique est ensuite séchée, filtrée et évaporée sous pression réduite. Le composé attendu est isolé sous forme d'un solide brun.
*Point de fusion : 84-85°C*

### STADE B : (Z)-N-[3-(Diméthylamino)propyl]-5-chloro-2,3-dihydro-3-(hydroxy-2-thiénylméthylène)-2-oxo-1H-indole-1-carboxamide, chlorhydrate

A une solution placée à 0°C contenant 7,44 mmoles du composé obtenu au stade précédent et 186 mg de 4-N,N-diméthylaminopyridine dans 5 ml de diméthylformamide sont ajoutés, sous atmosphère d'argon, 2,10 ml de triéthylamine et 7,44 mmoles de chlorure de 2-thénoyle. Le mélange réactionnel est laissé sous agitation à température ambiante pendant 3 heures. Après addition de 4 ml de méthanol puis 4 ml d'acide chlorhydrique 37 %, le mélange est encore agité une heure à température ambiante puis filtré. Le solide jaune obtenu est lavé à l'eau glacée et séché, et conduit au produit attendu.
*Point de fusion : 197-198°C (dec.)*

### STADE C : (Z)-N-[3-(Diméthylamino)propyl]-5-chloro-2,3-dihydro-3-(hydroxy-2-thiénylméthylène)-2-oxo-1H-indole-1-carboxamide, sel de sodium

Une suspension contenant 2,49 mmoles du produit obtenu au stade précédent et 1,25 mmoles de Na₂CO₃ dans 70 ml de méthanol est maintenue sous agitation à température ambiante pendant 5 heures. Le milieu réactionnel est ensuite concentré sous pression réduite et filtré. Le précipité est lavé à l'eau glacée et séché. Le produit obtenu est à nouveau traité par Na₂CO₃ en milieu méthanolique à température ambiante pendant 30 mn. Après évaporation, lavage du résidu au méthanol et séchage, on obtient le produit attendu.
*Point de fusion : 211-212°C (dec.)*

### STADE D : (Z)-(5-Chloro-1,2-dihydro-2-oxo-1-{[3-(triméthylammonio)propyl] aminocarbonyl}-3H-indol-3-ylidène)-2-thiénylméthanolate

A une solution contenant 2,22 mmoles du composé obtenu au stade précédent dans 30 ml de méthanol est ajouté, sous atmosphère d'argon, 3,33 mmoles d'iodure de méthyle. Le mélange est laissé 3 heures à température ambiante. Le produit attendu précipitant sous la forme d'un solide jaune au fur et à mesure de l'avancement de la réaction, est isolé par filtration, lavé au méthanol et à l'éther, et séché.
*Point de fusion : 260-261°C (dec.)*

### STADE E : Chlorure de {3-{[(Z)-5-chloro-2,3-dihydro-3-(hydroxy-2-thiénylméthylène)-2-oxo-1H-indol-1-yle]carbonylamino}propyl} triméthylammonium

A une solution contenant 0,95 mmole de produit obtenu au stade précédent dans 7 ml de diméthylformamide, est ajouté 2,5 ml d'éther chlorhydrique 2N. Le mélange réactionnel est agité 10 minutes à température ambiante. La solution obtenue est ensuite jetée dans 100 ml d'éther. Le précipité jaune alors obtenu est immédiatement filtré, lavé abondamment à l'éther et séché.
*Point de fusion : 209-211°C*

### EXEMPLE 2 : {3-{{4-[bis(2-Chloroéthyl)amino]-L-phénylalanyl}amino}propyl} triméthylammonium, chlorhydrate

### STADE A : 1-{{N-tert-butyloxycarbonyl-4-[bis(2-chloroéthyl)amino]-L-phényl-alanyl}amino}-3-(diméthylamino)propane

A une solution contenant 1,32 mmoles de chlorhydrate de Melphalan dans 7 ml de méthanol sont successivement ajoutés, à température ambiante, 2,7 mmoles de triéthylamine et 1,98 mmoles de dicarbonate de di-*tert*-butyle. Le mélange est ensuite placé à 30-40°C. Dès que la solubilisation a lieu, la solution est laissée sous agitation 30 minutes à température ambiante puis est évaporée sous pression réduite. Le résidu obtenu est traité par une solution glacée d'acide chlorhydrique dilué (0,01 N) jusqu'à pH = 2. La solution est alors immédiatement extraite avec de l'acétate d'éthyle. La phase organique est ensuite séchée, filtrée et concentrée sous pression réduite. L'intermédiaire ainsi obtenu est repris par 10 ml de dichlorométhane. A cette solution sont successivement ajoutés 1,33 mmoles de 1-hydroxybenzotriazole et 1,33 mmoles de 3-(diméthylamino)propylamine. Une solution contenant 1,33 mmoles de dicyclohexylcarbodiimide dans 10 ml de dichlorométhane est ensuite additionnée au mélange précédent. Le milieu réactionnel est laissé sous agitation à température ambiante pendant 5 heures. L'urée formée est isolée par filtration. Le filtrat est ensuite extrait avec une solution de NaHCO₃ 1N puis lavé à l'eau. La phase organique est séchée, filtrée et évaporée sous pression réduite. Le résidu obtenu est ensuite purifié par chromatographie sur gel de silice (éluant : dichlorométhane/éthanol, 1/1 puis dichlorométhane/éthanollammoniaque, 50/49/1). Le composé attendu est isolé sous forme d'une huile qui cristallise.
*Point de fusion : 80-82°C (déc.)*

### STADE B : Iodure de {3-{{N-tert-butyloxycarbonyl-4-[bis(2-chloroéthyl)amino]-L-phénylalanyl}amino}propyl}triméthylammonium

A une solution contenant 0,61 mmole du composé décrit au stade A dans 5 ml d'éthanol est ajouté sous atmosphère inerte 0,92 mmole d'iodure de méthyle. Le mélange réactionnel est laissé trois heures à température ambiante puis est concentré sous pression réduite. Le résidu obtenu est repris par le minimum de méthanol puis est jeté dans une solution éthérée. Le produit attendu est isolé sous la forme d'un solide très hygroscopique par filtration, lavage à l'éther et séchage.
*Point de fusion : 139-142°C*

### STADE C : {3-{{4-[bis(2-Chloroéthyl)amino]-L-phénylalanyl}amino}propyl} triméthylammonium, chlorhydrate

0,148 mmole du produit obtenu au stade B est traité par 10 ml d'éthanol chlorhydrique 2N à température ambiante pendant deux heures. La solution est ensuite évaporée sous pression réduite. Le résidu obtenu est solubilisé dans 50 ml de méthanol et passé sur résine pendant quelques minutes. Les fractions méthanoliques sont évaporées sous pression réduite. Le résidu obtenu est repris par le minimum de méthanol et est jeté dans une solution éthérée. Le produit attendu est isolé sous la forme d'un solide blanc-beige très hygroscopique par filtration, lavage à l'éther et séchage.
*Point de fusion : 115-120°C*
*Pouvoir rotatoire : [α]*_{*D*}^{*25*} *=+ 49,2° (c = 1,04 %, HCl 1N)*

### EXEMPLE 3 : Iodure de {3-{{4-[4-[bis(2-chloroéthyl)amino]phényl]butanoyl} amino}propyl}triméthylammonium

### STADE A : N-[3-(Diméthylamino)propyl]-4-{4-[bis(2-chloroéthyl)amino]phényl} butyramide

A une solution contenant 1,61 mmoles de chlorambucil dans 5 ml de dichlorométhane est ajouté, sous atmosphère inerte à 0°C, 1,25 ml de chlorure de thionyle. Le mélange réactionnel est laissé sous agitation à 4°C pendant 16 heures puis l'excès de SOCl₂ est évaporé sous pression réduite. Le résidu obtenu est repris par 10 ml de dichlorométhane. A cette solution est ajoutée, à 0°C et sous atmosphère inerte, 1,61 mmoles de 3-(diméthylamino)propylamine solubilisée dans 10 ml de dichlorométhane. Le mélange est ensuite agité à température ambiante pendant 1 heure. Au bout de ce temps, une seconde addition de 1,61 mmoles de diamine est réalisée. Après 4 heures d'agitation, le milieu réactionnel est évaporé sous pression réduite. Après neutralisation par une solution de NaHCO₃ 1N, la phase aqueuse est extraite plusieurs fois au dichlorométhane. Les différentes phases organiques sont regroupées, lavées à l'eau jusqu'à neutralité, séchées, filtrées et évaporées sous pression réduite. Le résidu obtenu est purifié par chromatographie sur gel de silice (éluant : gradient d'éthanol dans le dichlorométhane allant de 0 à 50 % puis pour finir utilisation de l'éluant : dichlorométhane/éthanol/ammoniaque, 50/49/1). Le produit attendu est ainsi obtenu sous la forme d'une huile.

### STADE B : Iodure de {3-{{4-[4-[bis(2-chloroéthyl)amino]phényl]butanoyl} amino}propyl}triméthylammonium

A une solution contenant 1,34 mmoles du composé obtenu au stade précédent dans 7 ml d'éthanol est ajouté, sous atmosphère inerte, 1,01 mmole d'iodure de méthyle. Le mélange est agité à température ambiante pendant 3 heures puis est évaporé sous pression réduite. L'huile obtenue est reprise par le minimum de méthanol. Cette solution est alors jetée dans 150 ml d'éther et agitée à 0°C pendant 1 heure. Le précipité formé est ensuite filtré. Après lavage à l'éther et séchage, le produit attendu est obtenu sous la forme d'un solide beige très hygroscopique.
*Point de fusion : 118-120°C (dec.)*

### EXEMPLE 4 : Chlorure de 2-(N,N,N-triméthylammonioacétamido)-2-déoxy-α,β-D-glucopyranose

### STADE A : 2-Chloroacétamido-2-déoxy-α,β-D-glucopyranose

A une solution refroidie à 0°C contenant 23,2 mmoles de chlorhydrate de glucosamine et de 40 mmol de K₂CO₃ dans 40 ml d'eau distillée, sont ajoutées goutte à goutte 46,4 mmoles de chlorure de chloroéthanoyle. L'agitation est poursuivie 1 heure. Après évaporation sous pression réduite de la solution aqueuse, le solide obtenu est lavé plusieurs fois à l'éthanol. La phase éthanolique est alors concentrée sous pression réduite jusqu'à précipitation d'un solide blanc. Après avoir été suffisamment refroidie à 0°C, la solution est filtrée. Le solide blanc obtenu est trituré à l'acétone et séché, et conduit au produit attendu après recristallisation dans l'éthanol.
*Point de fusion : 183-185°C*

### STADE B : Chlorure de 2-(N,N,N-triméthylammonioacétamido)-2-déoxy-α,β-D-glucopyranose

9,8 mmoles du composé obtenu au stade précédent et 10 ml d'une solution éthanolique de triéthylamine 4M sont placées sous atmosphère inerte pendant 3 jours à 40°C. Le produit attendu est obtenu par filtration du précipité formé suivie d'un lavage à l'éthanol, à l'éther et séchage.
*Point de fusion : 240-242°C*

### EXEMPLE 5 : Chlorure de 2-(pyridinioacétamido)-2-déoxy-α,β-D-glucopyranose

9,8 mmoles du composé obtenu au stade A de l'exemple 4 sont placées dans 50 ml de pyridine sous atmosphère inerte, à 40°C, pendant 3 jours. La pyridine est alors évaporée sous vide et le produit attendu est obtenu par lavage à l'éthanol, à l'éther et séchage.
*Point de fusion : 223-225°C*

### EXEMPLE 6 : Iodure de {3-[(4-hydroxy-2-méthyl-1,1-dioxide-2H-1,2-benzothiazin-3-yl)carboxamido]propyl}triméthylammonium

3,39 moles du *N*-[3-(diméthylamino)propyl]-4-hydroxy-2-méthyl-1,1-dioxo-2*H*-1,2-benzothiazin-3-yl]carboxamide sont chauffées sous argon à 80°C pendant 24 heures en présence de 3 ml d'iodométhane. Après refroidissement, le précipité obtenu est filtré, lavé à l'acétone et séché pour conduire au produit attendu.
*Point de fusion : 220-222°C (déc.)*

### EXEMPLE 7 : Iodure de 2-[(4-hydroxy-2-méthyl-1,1-dioxo-2H-1,2-benzothiazin-3-yl)carboxamido]-N-méthylpyridinium

Le produit attendu est obtenu par réaction du Piroxicam avec de l'iodométhane selon le procédé décrit dans l'exemple 6.

### EXEMPLE 8 : Bromure de [15]ane-N5-(N-3-propyl)triéthylammonium, chlorhydrate marqué au Technétium

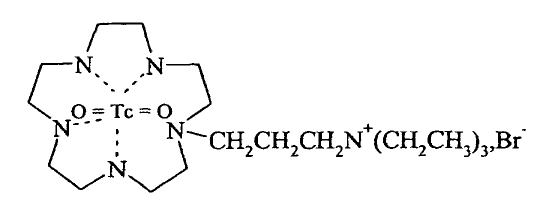

### STADE A : Bromure de [15]ane-N5-(N-3-propyl)triéthylammonium, chlorhydrate

A 10 mmoles de [15ane]-N5^{(*)} en solution dans 50 ml d'eau désionisée, sont ajoutées 10 mmoles de bromure de (3-bromopropyl)triéthylammonium. Après chauffage à 90°C pendant 12 heures sous atmosphère inerte, l'eau est évaporée. Le solide huileux est lavé deux fois au dichlorométhane, puis dissous dans 100 ml d'éthanol. Le traitement par 4 ml d'HCl 10N ajoutés, goutte à goutte, tout en refroidissant le ballon à 0°C, donne un précipité blanc, floconneux qui est filtré, lavé à l'alcool puis à l'éther et séché, et conduit au produit attendu.
*Point de fusion : > 200°C (déc.)*

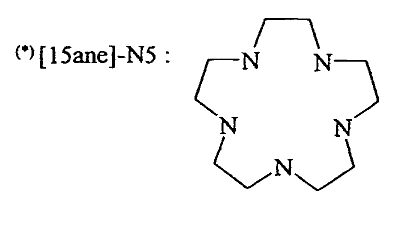

### STADE B : Bromure de [15]ane-N5-(N-3-propyl)triéthylammonium, chlorhydrate marqué au Technétium

Le marquage par le Technétium du composé obtenu au stade A se fait au sein d'un flacon stérile sous vide, d'une contenance de 15 ml, dans lequel on introduit :
- une solution de 7,5 mmoles du produit obtenu au stade A dans 1 ml de sérum physiologique,
- le pertechnétate de sodium (^{99m}TcO₄⁻, 25 mCi ; 925 MBq) en solution dans 1 ml de sérum physiologique ; le flacon est chauffé 5 minutes à 85°C (bain métallique),
- une solution aqueuse désoxygénée de SnCl₂,2H₂O (9 mmoles), préparée extemporanément.
Le marquage s'effectue par chauffage 30 minutes à 85°C.

### ETUDE PHARMACOLOGIQUE DES DERIVES DE L'INVENTION

### Etude pharmacocinétique : étude de la distribution tissulaire

Cette étude a été réalisée avec des molécules marquées au ¹⁴C. L'étude de la distribution tissulaire a été réalisée par mesure directe de la radioactivité sur des coupes de corps entier selon la méthode suivante : des rats mâles de souche Sprague-Dawley ont reçu par voie intraveineuse ou par voie orale une dose de molécule marquée. Les animaux ont ensuite été sacrifiés à des temps allant de 5 mn à 24 h. par inhalation d'éther et congelés dans l'azote liquide.

Des coupes ont alors été préparées avec un cryomicrotome et après dessiccation, la répartition de la radioactivité a été mesurée à l'aide d'un analyseur d'images.

Les résultats obtenus avec les dérivés de l'invention montrent que ces composés ont un tropisme renforcé pour les tissus cartilagineux.

Pour les composés des exemples 4 et 5, en dehors du rein, organe d'élimination fixant des quantités importantes de radioactivité dès les premières minutes suivant l'injection, le cartilage, et à moindre degré la peau, sont les seules cibles. Lorsque la même étude est réalisée avec la glucosamine non fonctionnalisée, le foie est l'organe cible prépondérant.

Pour le composé de l'exemple 6, le cartilage présente une affinité beaucoup plus élevée que les tissus environnants. Le maximum de fixation est obtenu 5 mn après injection. Lorsque cette étude est réalisée après administration par voie orale de la molécule marquée, il est également observé une très forte affinité pour le cartilage.

Le composé de l'exemple 8, quant à lui, se concentre de façon élevée dans les tissus cartilagineux 10 mn après injection.

## Revendications

1. Composés de formule (Ia) ou (Ib) : 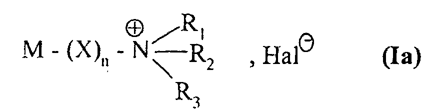 dans laquelle :
M représente une molécule utilisable pour la thérapie ou le diagnostic des pathologies dues à une atteinte du cartilage,
R₁, R₂, R₃, identiques ou différents, représentent un groupement alkyle (C₁-C₆) linéaire ou ramifié,
ou R₁, R₂, R₃ pris ensemble avec l'atome d'azote qui les porte forment un hétérocycle azoté saturé ou insaturé,
X représente une chaîne alkylène (C₁-C₆) linéaire ou ramifiée dans laquelle un ou plusieurs groupements -CH₂- sont éventuellement remplacés par un atome de soufre, d'oxygène, un groupement -NR- (dans lequel R représente un groupement alkyle (C₁-C₆) linéaire ou ramifié), un groupement -CO-, un groupement -CO-NH-, un groupement -CO₂-, un groupement -SO- ou un groupement -SO₂-,
n représente 0 ou 1,
Hal représente un atome d'halogène,
ou, 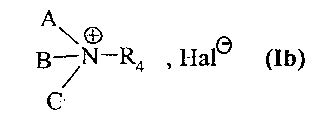
R₄ représente un groupement alkyle (C₁-C₆) linéaire ou ramifié,
Hal représente un atome d'halogène, 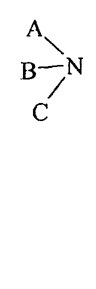
représente une molécule utilisable pour la thérapie ou le diagnostic des pathologies dues à une atteinte du cartilage, étant entendu que l'atome d'azote peut être éventuellement inclus dans un système hétérocyclique azoté saturé ou insaturé, ou engagé dans une double liaison.

2. Composé de formule (I) selon la revendication 1 **caractérisé en ce que** la molécule M ou 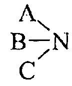 utilisable pour la thérapie des pathologies dues à une atteinte du cartilage est un antiinflammatoire, un analgésique, un antiarthrosique, un antiarthritique, ou un antitumoral spécifique.

3. Composé de formule (I) selon la revendication 1 tel qu'il est représenté pour la formule (Ia₁) : 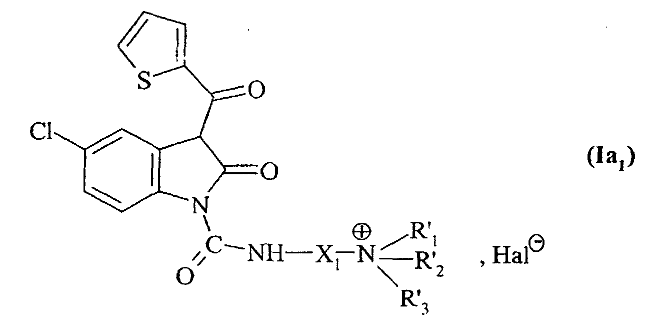 dans laquelle :
X₁ représente un groupement alkylène (C₁-C₆) linéaire ou ramifié,
R'₁, R'₂, R'₃, identiques ou différents, représentent un groupement alkyle (C₁-C₆) linéaire
ou ramifié,
Hal représente un atome d'halogène.

4. Composé de formule (I) selon la revendication 1 tel qu'il est représenté par la fonnule (Ia₂) : 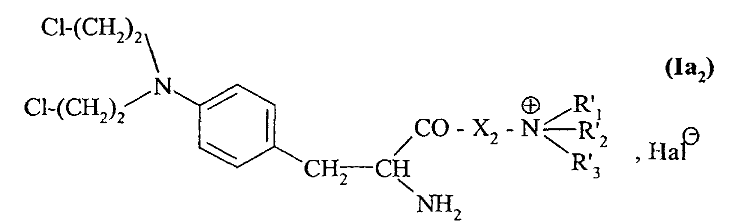 dans laquelle :
X₂ représente un groupement -NH-(CH₂)ₘ- dans lequel m représente un nombre entier compris entre 1 et 5 inclus,
R'₁, R'₂, R'₃, identiques ou différents, représentent un groupement alkyle (C₁-C₆) linéaire ou ramifié,
Hal représente un atome d'halogène.

5. Composé de formule (I) selon la revendication 1 tel qu'il est représenté par la formule (Ia₃) : 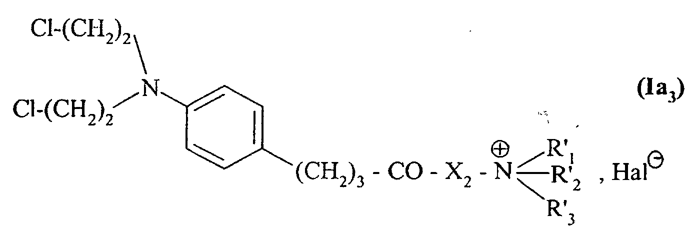 dans laquelle :
X₂ représente un groupement -NH-(CH₂)ₘ- dans lequel m représente un nombre entier compris entre 1 et 5 inclus,
R'₁, R'₂, R'₃, identiques ou différents, représentent un groupement alkyle (C₁-C₆) linéaire ou ramifié,
Hal représente un atome d'halogène.

6. Composé de formule (I) selon la revendication 1 tel qu'il est représenté par la formule (Ia₄) : 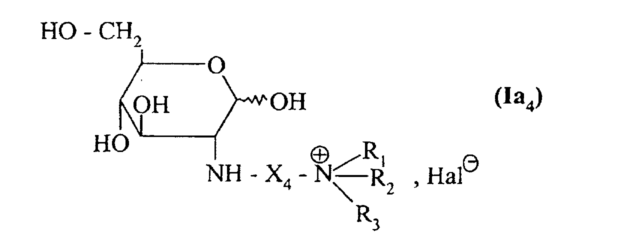 dans laquelle :
X₄ représente un groupement -CO-(CH₂)ₘ- dans lequel m représente un nombre entier compris entre 1 et 5 inclus,
R₁, R₂, R₃, identiques ou différents, représentent un groupement alkyle (C₁-C₆) linéaire ou ramifié,
ou R₁, R₂, R₃ pris ensemble avec l'atome d'azote qui les porte forment un hétérocycle azoté saturé ou insaturé,
Hal représente un atome d'halogène.

7. Composé de formule (I) selon la revendication 1 tel qu'il est représenté par la formule (Ib₁): 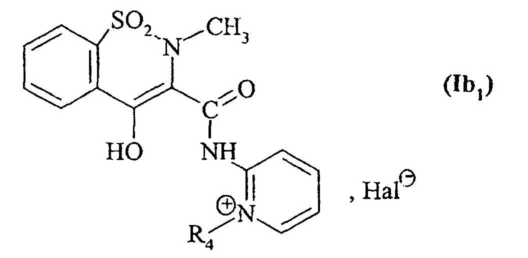 dans laquelle :
R₄ représente un groupement alkyle (C₁-C₆) linéaire ou ramifié,
Hal représente un atome d'halogène.

8. Composé de formule **(I)** selon la revendication 1 tel qu'il est représenté par la formule (Ib₂) : 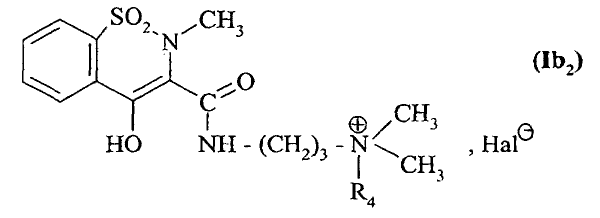 dans laquelle:
R₄ représente un groupement alkyle (C₁-C₆) linéaire ou ramifié,
Hal représente un atome d'halogène.

9. Composé de formule (I) selon la revendication 1 tel qu'il est représenté par la formule (Ia₅) : 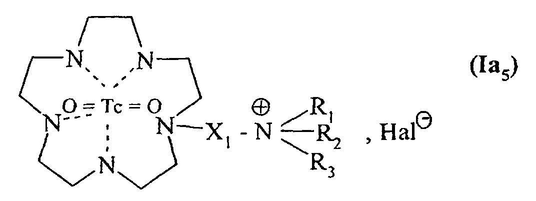 dans laquelle :
X₁ représente un groupement alkylène (C₁-C₆) linéaire ou ramifié,
R₁, R₂, R₃, identiques ou différents, représentent un groupement alkyle (C₁-C₆) linéaire ou ramifié,
ou R₁, R₂, R₃ pris ensemble avec l'atome d'azote qui les porte forment un hétérocycle azoté saturé ou insaturé,
Hal représente un atome d'halogène.

10. Procédé de préparation des composés de fonnule (Ia₁) selon la revendication 3 **caractérisé en ce qu'**ils sont obtenus à partir du 5-chloro-2,3-dihydro-2-oxo-1*H*-indole-1-carboxylate de 4-nitrophényle que l'on fait réagir avec une amine de formule (**II**) : 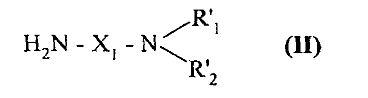 dans laquelle X₁, R'₁ et R'₂ sont tels que définis dans la revendication 3,
pour conduire au composé de formule (**III**) : 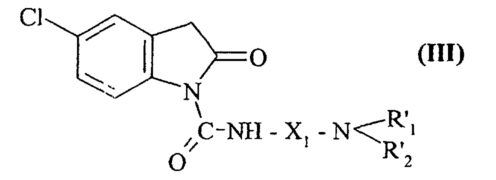 dans laquelle X₁, R'₁ et R'₂ sont tels que définis précédemment,
que l'on soumet à l'action du chlorure de 2-thénoyle en milieu basique sous atmosphère inerte, puis à un traitement acide,
pour conduire au composé de formule (**IV**) : 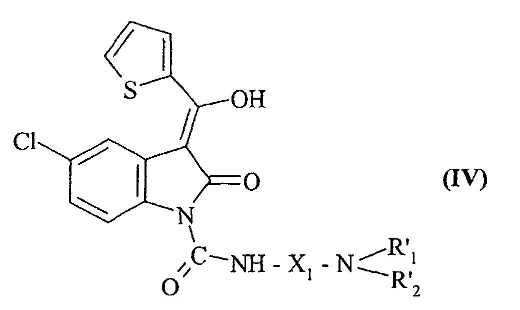 dans laquelle X₁, R'₁, et R'₂ sont tels que définis précédemment,
qui est transformé en sel de sodium correspondant,
puis qui subit l'action d'un halogénure d'alkyle (C₁-C₆) linéaire ou ramifié de formule R'₃Hal (dans laquelle R'₃ est tel que défini précédemment et Hal représente un atome d'halogène),
pour conduire au composé de formule (**V**) : 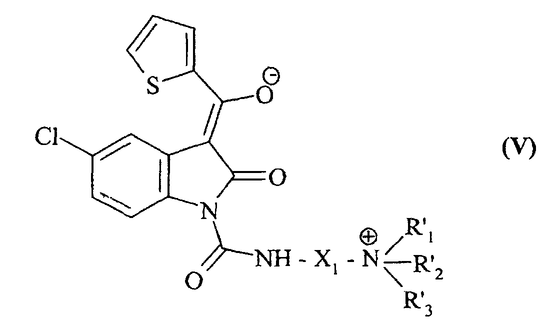 dans laquelle X₁, R'₁, R'₂ et R'₃ sont tels que définis précédemment,
qui, en milieu chlorhydrique, conduit au composé de formule (Ia₁), que l'on purifie le cas échéant.

11. Procédé de préparation des composés de formule (Ia₂) selon la revendication 4 **caractérisé en ce qu'**ils sont obtenus à partir du Melphalan dont la fonction amine a été préalablement protégée par un groupement *tert*-butoxycarbonyle (Boc) avec une amine de formule (**VI**) en présence d'un réactif de couplage peptidique : 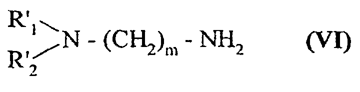 dans laquelle R'₁, R'₂ et m sont tels que définis dans la revendication 4,
pour conduire au composé de formule (**VII**) : 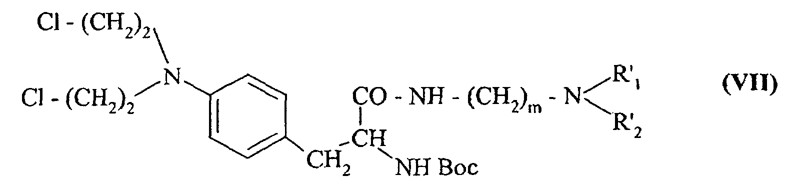 dans laquelle m, R'₁ et R'₂ sont tels que définis précédemment,
que l'on soumet à l'action d'un halogénure d'alkyle (C₁-C₆) linéaire ou ramifié de formule R'₃Hal (dans laquelle R'₃ est tel que défini précédemment et Hal représente un atome d'halogène), puis à un traitement chlorhydrique,
pour conduire au composé de formule (Ia₂) que l'on purifie le cas échéant.

12. Procédé de préparation des composés de formule (Ia₃) selon la revendication 5 **caractérisé en ce qu'**ils sont obtenus à partir du Chlorambucil dont on transforme la fonction acide en chlorure d'acide correspondant,
puis que l'on fait réagir avec une amine de formule (**VI**), en présence ou non d'un réactif de couplage peptidique : 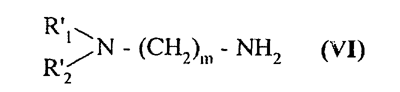 dans laquelle R'₁, R'₂ et m sont tels que définis dans la revendication 5,
pour conduire au composé de formule (**VIII**) : 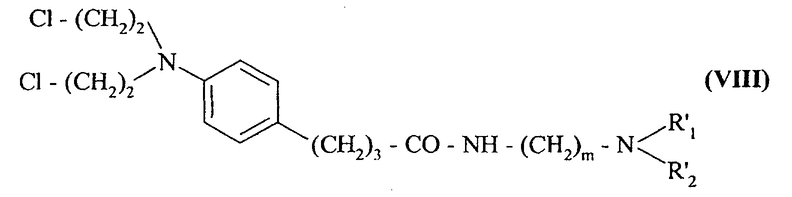 dans laquelle m, R'₁ et R'₂ sont tels que définis précédemment,
que l'on soumet à l'action d'un halogénure d'alkyle (C₁-C₆) linéaire ou ramifié de formule R'₃Hal (dans laquelle R'₃ est tel que défini précédemment et Hal représente un atome d'halogène),
pour conduire au composé de formule (Ia₂) que l'on purifie le cas échéant.

13. Procédé de préparation des composés de formule (Ia₄) selon la revendication 6 **caractérisé en ce qu'**ils sont obtenus par réaction de la Glucosamine avec un chlorure d'acide de formule (**IX**) :
Cl - (CH₂)ₘ - CO - Cl (**IX**)
dans laquelle m est tel que défini dans la revendication 6,
pour conduire au composé de formule (**X**) : 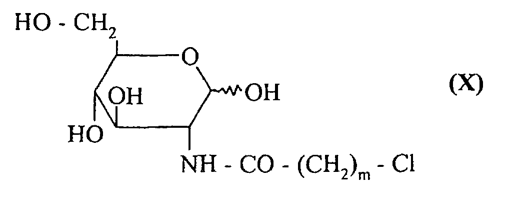 dans laquelle m est tel que défini précédemment,
que l'on condense sur une amine de formule (**XI**) : 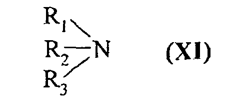 dans laquelle R₁, R₂, R₃ sont tels que définis dans la revendication 6,
pour conduire au composé de formule (Ia₄) que l'on purifie le cas échéant et dont on sépare éventuellement les isomères selon une technique classique de séparation.

14. Procédé de préparation des composés de formule (Ia₅) selon la revendication 9 **caractérisé en ce qu'**ils sont obtenus à partir du composé de formule (**XII**): 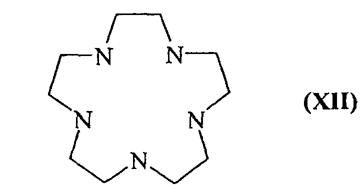 sur lequel on fait réagir un halogénure d'halogénoalkylamrnonium de formule (**XIII**) : 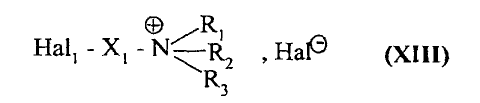 dans laquelle X₁, R₁, R₂, R₃ sont tels que définis dans la revendication 9, Hal et Hal₁, identiques ou différents, représentent des atomes d'halogène,
pour conduire au composé de formule (**XIV**) : 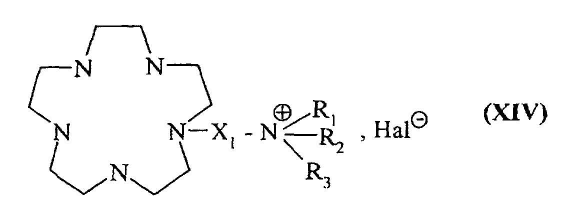 dans laquelle X₁, R₁, R₂, R₃ et Hal sont tels que définis précédemment,
que l'on fait réagir avec du pertechnéate de sodium en présence de chlorure d'étain, pour conduire au composé de formule (Ia₅) que l'on purifie le cas échéant.

15. Procédé de préparation des composés de formule (Ib₁) selon la revendication 7 **caractérisé en ce qu'**ils sont obtenus à partir du Piroxicam sur lequel on fait réagir un halogénure d'alkyle (C₁-C₆) linéaire ou ramifié, que l'on purifie le cas échéant.

16. Procédé de préparation des composés de formule (Ib₂) selon la revendication 8 **caractérisé en ce qu'**ils sont obtenus à partir de l'amine correspondante sur laquelle on fait réagir un halogénure d'alkyle (C₁-C₆) linéaire ou ramifié, que l'on purifie le cas échéant.

17. Composition pharmaceutique contenant comme principe actif un composé selon l'une quelconque des revendications 1 à 9 seul ou en combinaison avec un ou plusieurs excipients ou véhicules inertes non toxiques, pharmaceutiquement acceptables.

18. Composition pharmaceutique selon la revendication 17 contenant un composé selon l'une des revendications 1 à 9 utile pour le traitement des pathologies dues à une atteinte du cartilage.

19. Composition pharmaceutique selon la revendication 17 contenant un composé selon l'une des revendications 1 ou 9 utile à titre de réactif de diagnostic capable de révéler une pathologie du cartilage ou un métabolisme.

## Patentansprüche

1. Verbindungen der Formel (Ia) oder (Ib) : 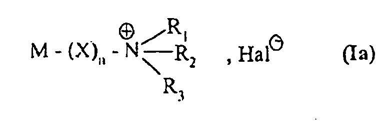 in der:
M ein Molekül darstellt, das für die Therapie oder Diagnostik von durch eine Knorpelschädigung bedingten Krankheiten verwendbar ist,
R₁, R₂, R₃, die gleich oder verschieden sind, eine lineare oder verzweigte (C₁-C₆)-Alkylgruppe darstellen, oder R₁, R₂, R₃ zusammen mit dem sie tragenden Stickstoffatom einen gesättigten oder ungesättigten Stickstoffheterocyclus bilden,
X eine lineare oder verzweigte (C₁-C₆)-Alkylenkette darstellt, in der eine oder mehrere Gruppen -CH₂- ggf. durch ein Schwefelatom, ein Sauerstoffatom, eine Gruppe - NR- (in der R eine lineare oder verzweigte (C₁-C₆)-Alkylgruppe darstellt), eine Gruppe -CO-, eine Gruppe - CO-NH-, eine Gruppe -CO₂-, eine Gruppe -SO- oder eine Gruppe -SO₂- ersetzt sind,
n 0 oder 1 darstellt,
Hal ein Halogenatom darstellt,
oder 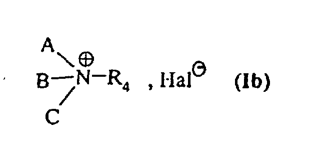
R4 eine lineare oder verzweigte (C₁-C₆)-Alkylgruppe darstellt,
Hal ein Halogenatom darstellt, 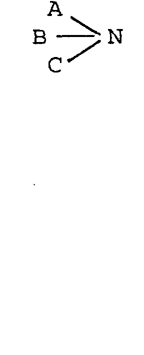
ein Molekül darstellt, das für die Therapie oder Diagnostik von durch eine Knorpelschädigung bedingten Krankheiten verwendbar ist, wobei gilt, daß das Stickstoffatom ggf. in ein gesättigtes oder ungesättigtes heterocyclisches Stickstoffsystem eingeschlossen sein kann oder in eine Doppelbindung eingebunden sein kann.

2. Verbindung der Formel (I) nach Anspruch 1, **dadurch gekennzeichnet, daß** das Molekül M oder 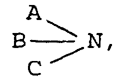 das für die Therapie von durch eine Knorpelschädigung bedingten Krankheiten verwendbar ist, ein entzündungshemmendes Mittel, ein Analgetikum, ein Antiarthrotikum, ein Antiarthritikum oder ein spezifisches antitumoral wirkendes Mittel ist.

3. Verbindung der Formel (I) nach Anspruch 1, wie sie durch die Formel (Ia₁) dargestellt wird: 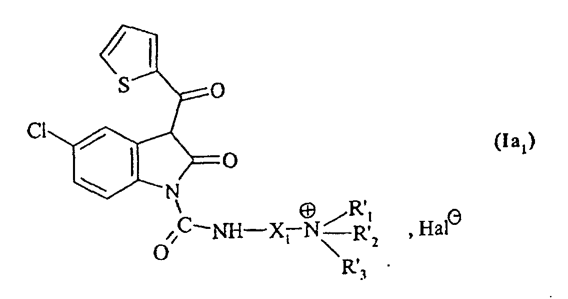 in der:
X₁ eine lineare oder verzweigte (C₁-C₆)-Alkylengruppe darstellt,
R'₁, R'₂, R', die gleich oder verschieden sind, eine lineare oder verzweigte (C₁-C₆)-Alkylgruppe darstellen,
Hal ein Halogenantom darstellt.

4. Verbindung der Formel (I) nach Anspruch 1, wie sie durch die Formel (Ia₂) dargestellt wird: 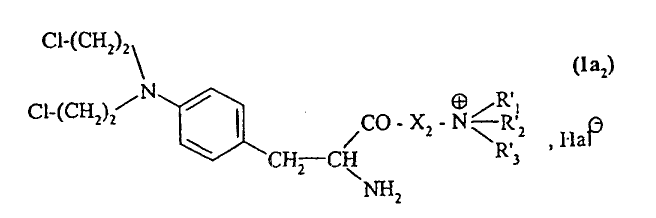 in der :
X₂ eine Gruppe -NH-(CH₂)ₘ- darstellt, in der m eine ganze Zahl zwischen einschließlich 1 und 5 ist,
R'₁, R'₂, R'₃, die gleich oder verschieden sind, eine lineare oder verzweigte (C₁-C₆)-Alkylgruppe darstellen,
Hal ein Halogenatom darstellt.

5. Verbindung der Formel (I) nach Anspruch 1, wie sie durch die Formel (Ia₃) dargestellt wird: 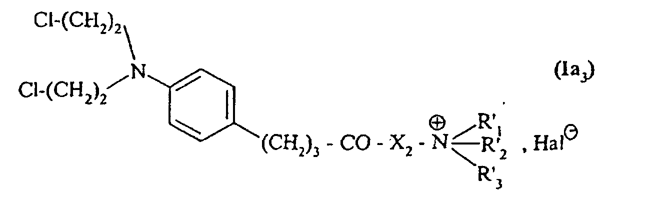 in der:
X₂ eine Gruppe -NH-(CH₂)ₘ- darstellt, in der m eine ganze Zahl zwischen einschließlich 1 und 5 darstellt,
R'₁, R'₂, R'₃, die gleich oder verschieden sind, eine lineare oder verzweigte (C₁-C₆)-Alkylgruppe darstellen,
Hal ein Halogenatom darstellt.

6. Verbindung der Formel (I) nach Anspruch 1, wie sie durch die Formel (Ia₄) dargestellt wird: 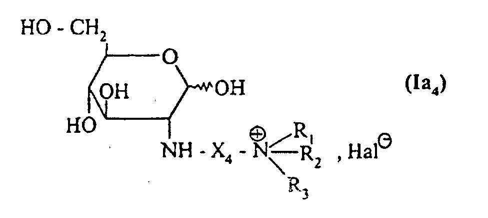 in der:
X₄ eine Gruppe -CO-(CH₂)ₘ- darstellt, in der m eine ganze Zahl zwischen einschließlich 1 und 5 darstellt,
R₁, R₂, R₃, die gleich oder verschieden sind, eine lineare oder verzweigte (C₁-C₆)-Alkylgruppe darstellen, oder R₁, R₂, R₃ zusammen mit dem sie tragenden Stickstoffatom einen gesättigten oder ungesättigten Stickstoffheterocyclus bilden,
Hal ein Halogenatom darstellt.

7. Verbindung der Formel (I) nach Anspruch 1, wie sie durch die Formel (Ib₁) dargestellt wird: 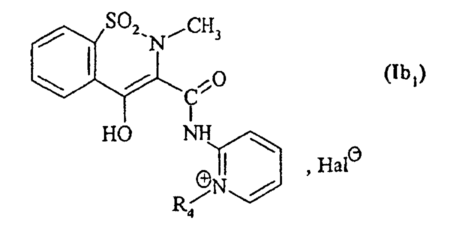 in der:
R₄ eine lineare oder verzweigte (C₁-C₆)-Alkylgruppe darstellt,
Hal ein Halogenatom darstellt.

8. Verbindung der Formel (I) nach Anspruch 1, wie sie durch die Formel (Ib₂) dargestellt wird: 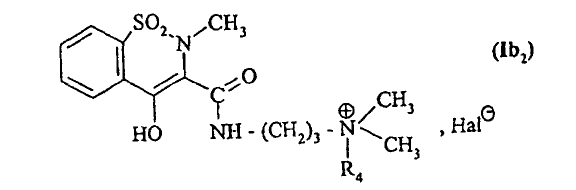 in der:
R4 eine lineare oder verzweigte (C₁-C₆)-Alkylgruppe darstellt,
Hal ein Halogenatom darstellt.

9. Verbindung der Formel (I) nach Anspruch 1, wie sie durch die Formel (Ia₅) dargestellt wird: 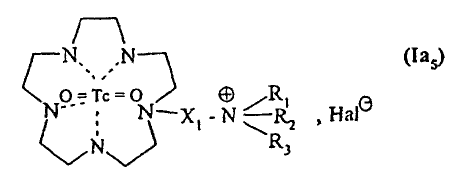 in der:
X₁ eine lineare oder verzweigte (C₁-C₆)-Alkylengruppe darstellt,
R₁, R₂, R₃, die gleich oder verschieden sind, eine lineare oder verzweigte (C₁-C₆)-Alkylgruppe darstellen oder R₁, R₂, R₃ zusammen mit dem sie tragenden Stickstoffatom einen gesättigten oder ungesättigten Stickstoffheterocyclus bilden,
Hal ein Halogenatom darstellt.

10. Verfahren zur Herstellung der Verbindungen der Formel (Ia₁) nach Anspruch 3, **dadurch gekennzeichnet, daß** sie aus dem 4-Nitrophenylester der 5-Chlor-2,3-dihydro-2-oxo-1*H*-indol-1-carbonsäure erhalten werden, die man mit einem Amin der Formel (II) reagieren läßt: 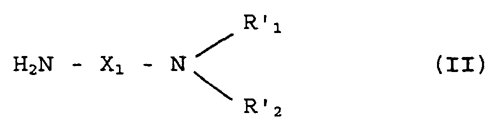 in der X₁, R'₁ und R'₂ von der in Anspruch 3 definierten Art sind,
um zu der Verbindung der Formel (III) zu führen: 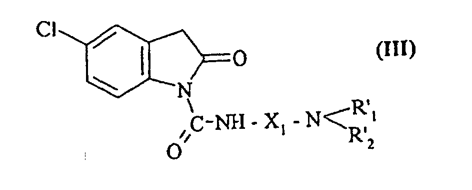 in der X₁, R'₁ und R'₂ von der oben definierten Art sind, die man der Einwirkung von 2-Thenoylchlorid in basischem Medium unter Inertatmosphäre und dann einer sauren Behandlung unterzieht,
um zu der Verbindung der Formel (**IV**) zu führen: 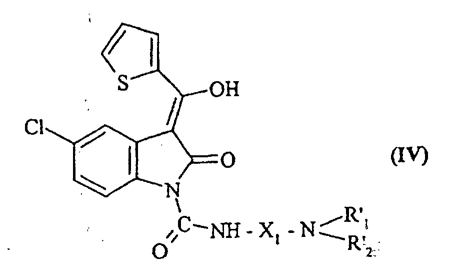 in der X₁, R'₁ und R'₂ von der oben definierten Art sind, die in das entsprechende Natriumsalz überführt wird und dann der Einwirkung eines linearen oder verzweigten (C₁-C₆)-Alkylhalogenids der Formel R'₃Hal (in der R'₃ von der oben definierten Art ist und Hal ein Halogenatom darstellt) ausgesetzt wird,
um zu der Verbindung der Formel (V) zu führen: 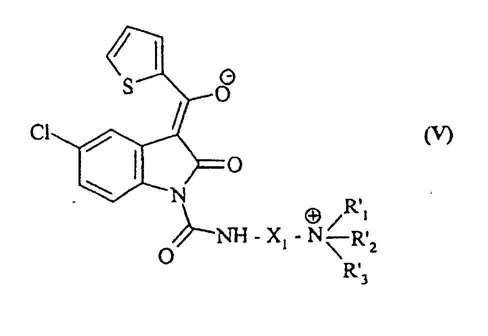 in der X₁, R'₁, R'₃ von der oben definierten Art sind, die in Hydrochlormedium zu der Verbindung der Formel (Ia₁) führt, die man ggf. reinigt.

11. Verfahren zur Herstellung von Verbindungen der Formel (Ia₂) nach Anspruch 4, **dadurch gekennzeichnet, daß** sie aus Melphalan, dessen Aminfunktion zuvor durch eine tert-Butoxycarbonyl-Gruppe (Boc) geschützt wurde, mit einem Amin der Formel (**VI**) : 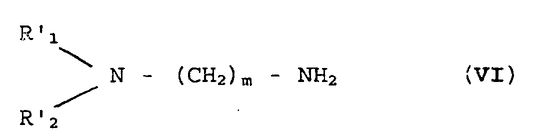 in der R'₁, R'₂ und m von der in Anspruch 4 definierten Art sind, in Gegenwart eines Peptidkopplungsreagenzes erhalten werden, um zur Verbindung der Formel (VII) zu führen: 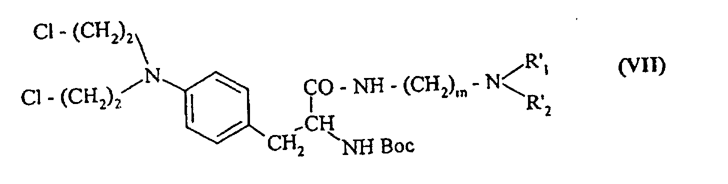 in der m, R'₁ und R'₂ von der oben definierten Art sind, die man der Einwirkung eines linearen oder verzweigten (C₁-C₆)-Alkylhalogenids der Formel R'₃Hal (in der R'₃ von der oben definierten Art ist und Hal ein Halogenatom darstellt) und dann einer Hydrochlorbehandlung aussetzt, um zur Verbindung der Formel (Ia₂) zu führen, die man ggf. reinigt.

12. Verfahren zur Herstellung der Verbindungen der Formel (Ia₃) nach Anspruch 5, **dadurch gekennzeichnet, daß** sie aus Chlorambucil erhalten werden, dessen saure Funktion man in das entsprechende Säurechlorid überführt, und daß man es dann in Gegenwart eines Peptidkopplungsreagenzes oder nicht mit einem Amin der Formel (**VI**) reagieren läßt: 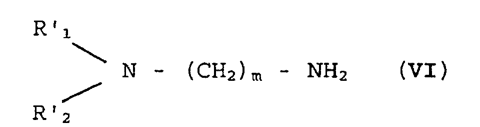 in der R'₁, R'₂ und m von der in Anspruch 5 definierten Art sind, um zur Verbindung der Formel (**VIII**) zu führen: 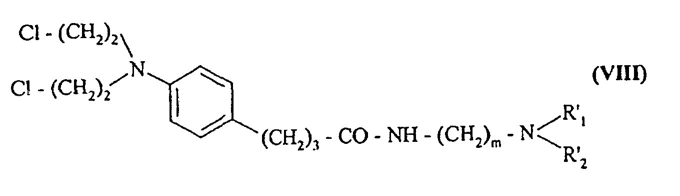 in der m, R'₁ und R'₂ von der oben definierten Art sind, die man der Einwirkung eines linearen oder verzweigten (C₁-C₆)-Alkylhalogenids der Formel R'₃Hal (in der R'₃ von der oben definierten Art ist und Hal ein Halogenatom darstellt) unterzieht, um zu der Verbindung der Formel (Ia₂) zu führen, die man ggf. reinigt.

13. Verfahren zur Herstellung der Verbindungen der Formel (Ia₄) nach Anspruch 6, **dadurch gekennzeichnet, daß** sie durch Reaktion von Glucosamin mit einem Säurechlorid der Formel (**IX**) erhalten werden:
Cl-(CH₂)ₘ-CO-Cl (**IX**)
in der m von der in Anspruch 6 definierten Art ist,
um zu der Verbindung der Formel (**X**) zu führen: 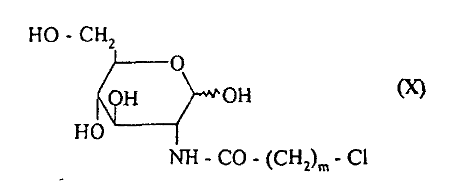 in der m von der oben definierten Art ist,
die man an einem Amin der Formel (XI) kondensiert: 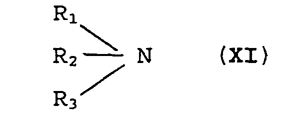 in der R₁, R₂, R₃ von der in Anspruch 6 definierten Art sind,
um zu der Verbindung der Formel (Ia₄) zu führen, die man ggf. reinigt und von der man ggf. die Isomere nach einer herkömmlichen Trenntechnik auftrennt.

14. Verfahren zur Herstellung der Verbindungen der Formel (Ia₅) nach Anspruch 9, **dadurch gekennzeichnet, daß** sie aus der Verbindung der Formel (**XII**) erhalten werden: 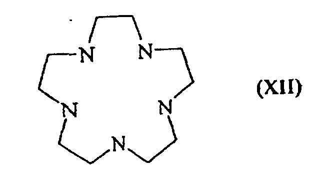 mit der man ein Halogenalkylammoniumhalogenid der Formel (XIII) reagieren läßt: 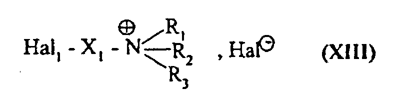 in der X₁, R₁, R₂, R₃ von der in Anspruch 9 definierten Art sind und Hal und Hal₁, die gleich oder verschieden sind, Halogenatome darstellen,
um zu der Verbindung der Formel (**XIV**) zu führen: 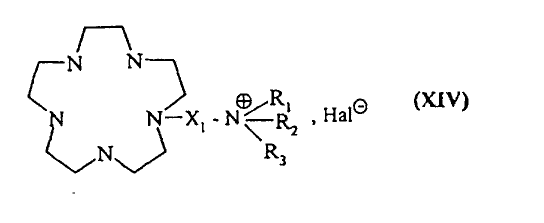 in der X₁, R₁, R₂, R₃ und Hal von der oben definierten Art sind,
das man in Gegenwart von Zinnchlorid mit Natriumpertechneat reagieren läßt,
um zu der Verbindung der Formel (Ia₅) zu führen, die man ggf. reinigt.

15. Verfahren zur Herstellung der Verbindungen der Formel (Ib₁) nach Anspruch 7, **dadurch gekennzeichnet, daß** sie aus Piroxicarn erhalten werden, mit dem man ein lineares oder verzweigtes (C₁-C₆)-Alkylhalogenid reagieren läßt, das man ggf. reinigt.

16. Verfahren zur Herstellung der Verbindungen der Formel (Ib₂) nach Anspruch 8, **dadurch gekennzeichnet, daß** sie aus dem entsprechenden Amin erhalten werden, mit dem man ein lineares oder verzweigtes (C₁-C₆)-Alkylhalogenid reagieren läßt, das man ggf. reinigt.

17. Pharmazeutische Zusammensetzung, die als Wirkstoff eine Verbindung nach einem der Ansprüche 1 bis 9 allein oder in Kombination mit einer bzw. einem oder mehreren pharmazeutisch verträglichen, nicht toxischen Grundmassen oder Trägern enthält.

18. Pharmazeutische Zusammensetzung nach Anspruch 17, die eine Verbindung nach einem der Ansprüche 1 bis 9 enthält, die für die Behandlung von durch eine Schädigung des Knorpels bedingten Krankheiten geeignet ist.

19. Pharmazeutische Zusammensetzung nach Anspruch 17, die eine Verbindung nach einem der Ansprüche 1 oder 9 enthält, die als Diagnosereagenz für den Nachweis einer Knorpel- oder Stoffwechselerkrankung geeignet ist.

## Claims

1. Compounds of the formula (Ia) or (Ib): 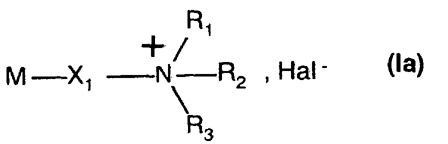 wherein:
M stands for a molecule useful in the therapy or diagnosis of pathologies due to cartilage damage,
R₁, R₂, R₃, identical or different, stand for a (C₁-C₆) linear or branched alkyl group, or R₁, R₂, R₃ taken together with the nitrogen atom which bears them form a saturated or unsaturated nitrogen heterocycle,
X stands for a (C₁-C₆) linear or branched alkylene chain in which one or more CH₂ groups are optionally substituted by a sulfur atom, an oxygen atom, a -NR- group ( in which R stands for a (C₁-C₆) linear or branched alkyl group), a -CO- group, a -CO-NH- group, a - CO₂- group, a -SO- group, a -SO₂- group,
n stands for 0 or 1,
Hal stands for a halogen atom,
or, 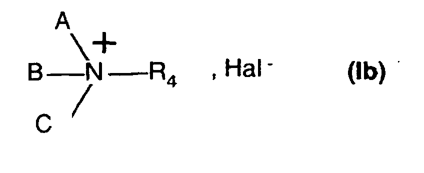
R₄ stands for a (C₁-C₆) linear or branched alkyl group,
Hal stands for a halogen atom, 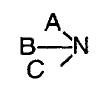
stands for a molecule useful in the therapy or diagnosis of pathologies due to cartilage damage, it being understood that the nitrogen atom can optionally be included in a saturated or unsaturated nitrogen heterocycle, or held within a double bond

2. Compound of formula (I) according to claim 1, **characterised in that** the molecule M or 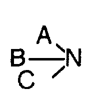 useful for the therapy of pathologies due to cartilage damage is an anti-inflammatory, an analgesic, an anti-arthrosis, an anti-arthritic or a specific anti-tumor agent.

3. Compound of formula (I) according to claim 1 as represented by the formula (Ia₁): 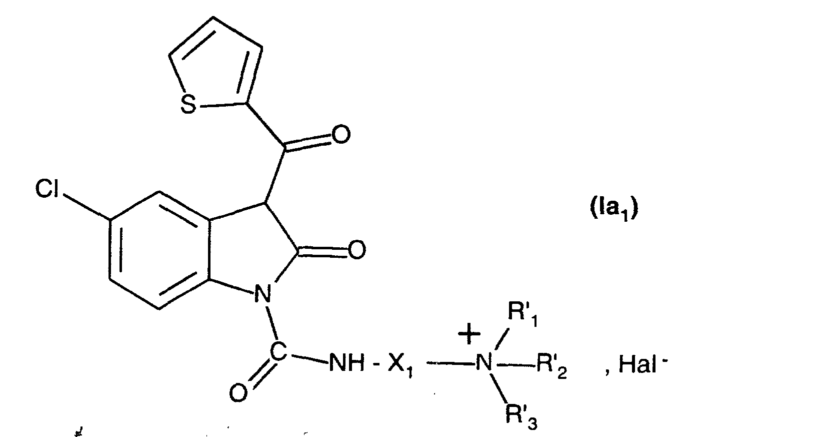 wherein:
X₁ stands for a (C₁-C₆) linear or branched alkylene group,
R'₁, R'₂, R'₃, identical or different, stand for a (C₁-C₆) linear or branched alkyl group , Hal stands for a halogen atom.

4. Compound of formula (I) according to claim 1 as represented by the formula (Ia₂) 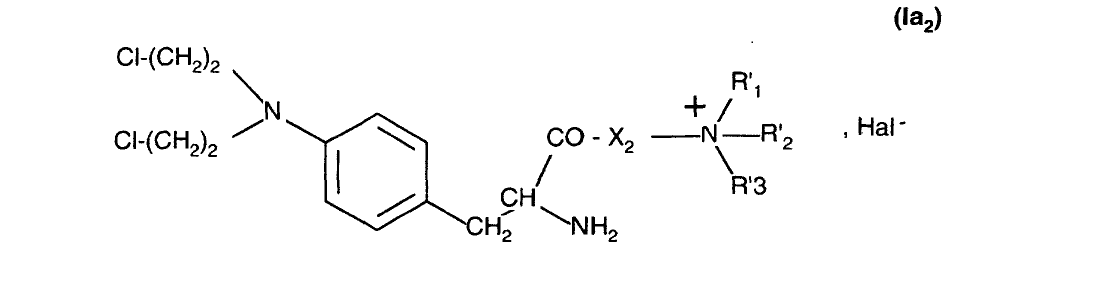 wherein:
X₂ stands for a -NH-(CH₂)ₘ- in which m stands for a whole number comprised between 1 and 5 inclusive,
R'₁, R'₂, R'₃, identical or different, stand for a (C₁-C₆) linear or branched alkyl group,
Hal stands for a halogen atom.

5. Compound of formula (I) according to claim 1 as represented by the formula (Ia₃) 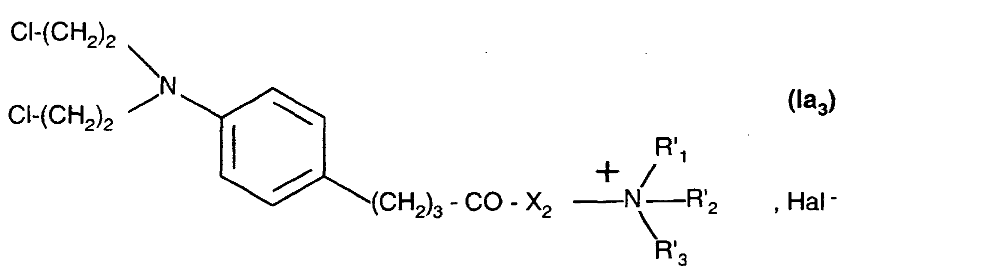 wherein:
X₂ stands for a -NH-(CH₂)- group in which m stands for a whole number comprised between 1 and 5 inclusive,
R'₁, R'₂, R'₃, identical or different stand for a (C₁-C₆) linear or branched alkyle group,
Hal stands for a halogen atom.

6. Compound of formula (I) according to claim 1 as represented by the formula (Ia₄): 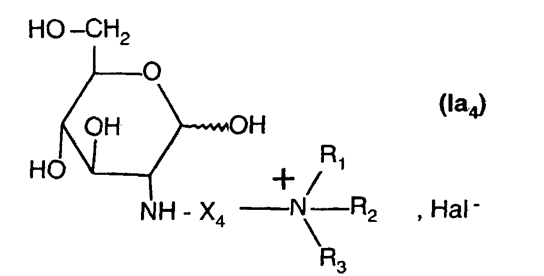 wherein:
X₄ stands for a -CO-(CH₂)ₘ- group in which m stands for a whole number comprised between 1 and 5 inclusive,
R₁, R₂, R₃, identical or different, stand for a (C₁-C₆) linear or branched alkyl group , or R₁, R₂, R₃, taken together with the nitrogen atom which bears them form a saturated or unsaturated nitrogen heterocycle, Hal stands for a halogen atom.

7. Compound of formula (I) according to claim 1 as represented by the formula (Ib₁): 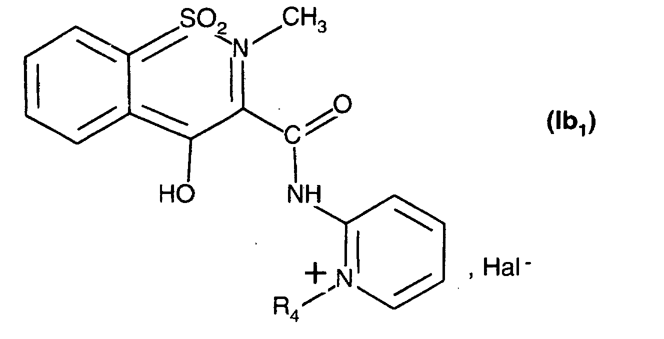 wherein:
R₄, stands for a linear or branched (C₁-C₆) alkyl group,
Hal stands for a halogen atom.

8. Compound of formula (I) according to claim 1 as represented by the formula (Ib₂): 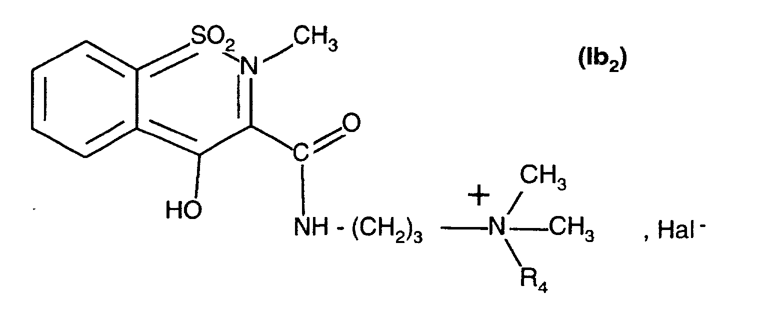 wherein:
R₄ stands for a (C₁-C₆) linear or branched alkyl group,
Hal stands for a halogen atom.

9. Compound of formula (I) according to claim 1 as represented by the formula (Ia₅): 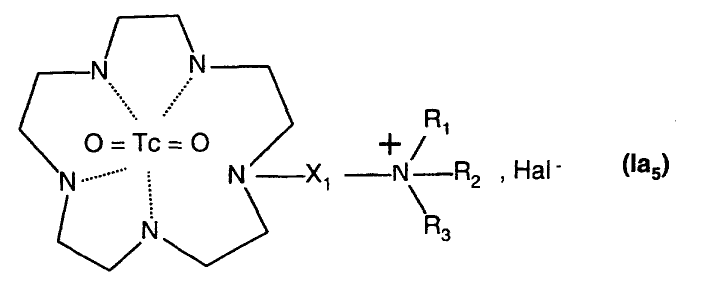 wherein:
X₁ stands for a (C₁-C₆) linear or branched alkylene group,
R₁, R₂, R₃, identical or different, stand for a (C₁-C₆) linear or branched alkyl group,
or R₁, R₂, R₃, taken together with the nitrogen atom which bears them form a saturated or unsaturated nitrogen heterocycle,
Hal stands for a halogen atom.

10. Process for the preparation of compounds of the formula (Ia₁) according to claim 3 **characterised in that** they are obtained from 4-nitrophenyl-5-chloro-2,3-dihydro-2-oxo-1H-indole-1-carboxylate reacted with an amine of formula (II): 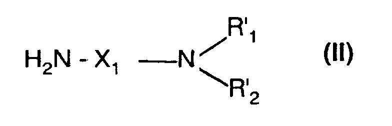 wherein X₁, R'₁ and R'₂ are as defined in claim 3, leading to the compound of formula (III) 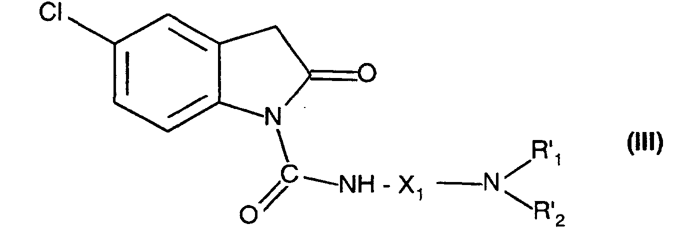 wherein X₁, R'₁ and R'₂ are as stated above, which is reacted with 2-thenoyl chloride in basic media under an inert atmosphere, then subjected to an acid treatment, leading to the compound of formula (IV): 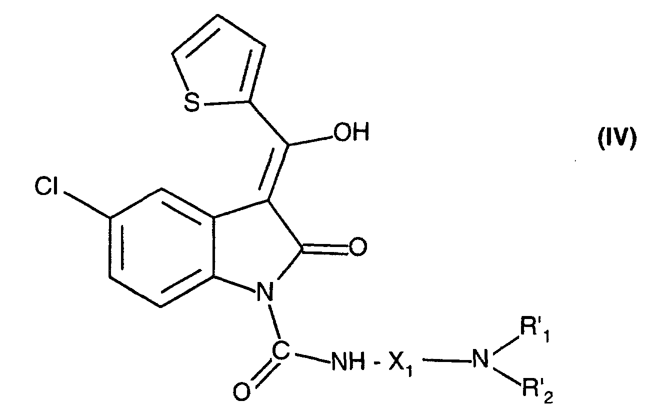 wherein X₁, R'₁ and R'₂ are as stated above, which is converted into its corresponding sodium salt, then subjected to the action of a (C₁-C₆) linear or branched alkyl halide of the formula R'₃Hal (wherein R'₃ is as stated above and Hal stands for a halogen atom) leading to the compound of formula (V): 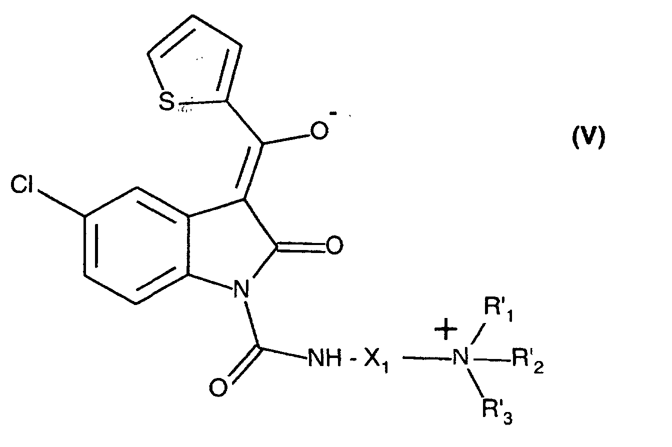 wherein X₁, R'₁, R'₂ and R'₃ are as stated above, which in a hydrochloric acid environment leads to the compound of the formula (Ia₁), which is optionally purified.

11. Process for the preparation of the compounds of formula (Ia₂) according to claim 4 **characterised in that** they are obtained from Melphalan in which the animo function has been previously protected by a tert-butoxycarbonyl (Boc) group with an amine of formula (VI) in the presence of a peptide coupling reagent: 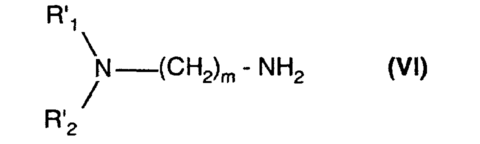 wherein R'₁, R'₂ and m as defined in claim 4, leading to the compound of formula (VII): 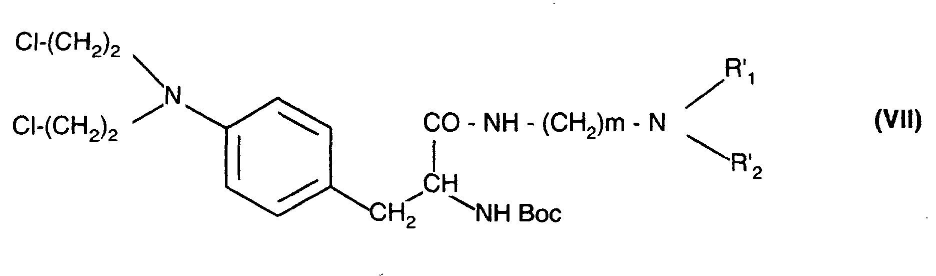 wherein m, R'1 and R'₂ are as stated above, which is subjected to the action of a (C₁-C₆) linear or branched alkyl halide of formula R'₃Hal (wherein R₃ is as stated above and Hal stands for a halogen atom), then subjected to a hydrochloric acid treatment leading to the compound of the formula (Ia₂) which is optionally purified.

12. Process for the preparation of the compounds of formula (Ia₃) according to claim 5 **characterised in that** they are obtained from Chlorambucil in which the acid function is converted into the corresponding acid chloride, which is then reacted with an amine of formula (VI), in the presence or absence of a peptide coupling reagent: 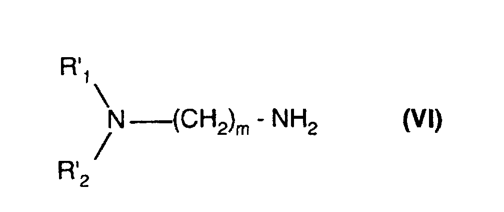 wherein R'₁ and R'₂ are as stated above in claim 5, leading to the compound of formula (VIII) 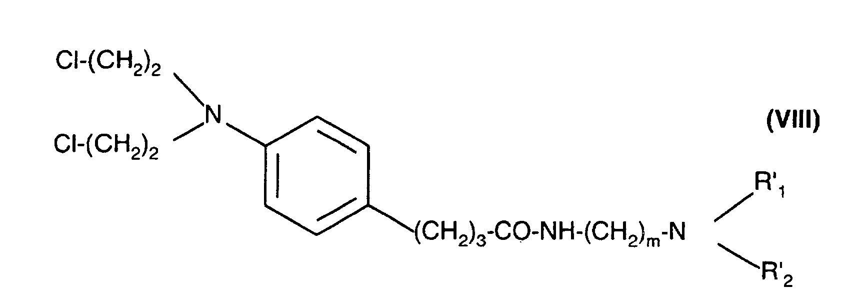 wherein m, R'₁ and R'₂ are as stated above, which is subjected to the action of a (C₁-C₆) linear or branched alkyl halide of formula R'₃Hal (wherein R'₃ is as stated above and Hal stands for a halogen atom), leading to the compounds of formula (Ia₂) which is optionally purified.

13. Process for the preparation of the compounds of formula (Ia₄) according to claim 6 **characterised in that** they are obtained by reaction of Glucosamine with an acid chloride of formula (IX):
Cl - (CH₂)ₘ - CO - Cl (**IX**)
wherein m is as stated in claim 6, leading to the compound of formula (X): 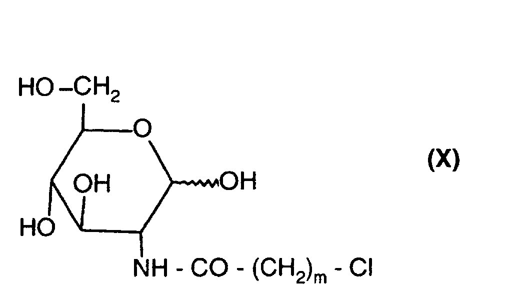 wherein m is stated as above, which is condensed on an amine of formula (XI): 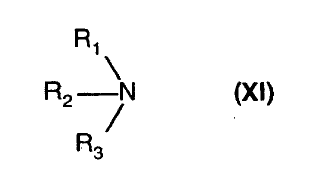 wherein R₁, R₂, R₃ are as defined in claim 6,
leading to the compound of formula (la₄) which is optionally purified and in which the isomers are optionally separated according to a classical technique of separation.

14. Process for the preparation of the compounds of formula (la₅) according to claim 9 **characterised in that** they are obtained from the compound of formula (XII): 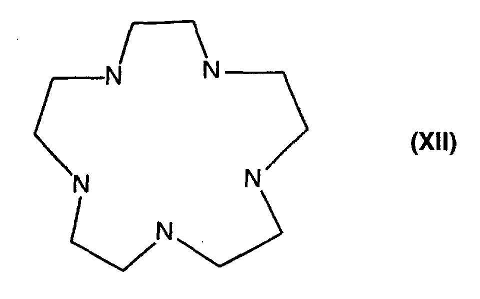 which is reacted with a halogenoalkylammonium halide of formula (XIII): 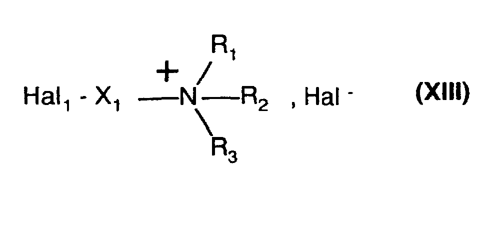 wherein X₁, R₁, R₂, R₃ and Hal are as stated above in claim 9, Hal and Hal₁, identical or different stand for halogen atoms, leading to the compound of formula (XIV): 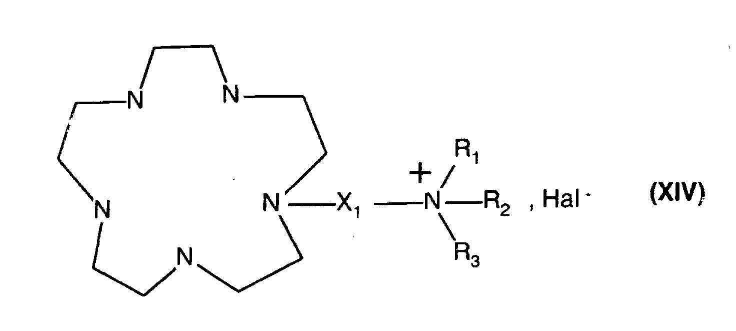 wherein X₁, R₁, R₂, R₃, and Hal are as stated above, which is reacted with sodium pertechneate in the presence of tin chloride leading to the compound of formula (Ia₅), which is then optionally purified.

15. Process for the preparation of the compounds of the formula (Ib₁) according to claim 7, **characterised in that** they are obtained from Piroxicam which is reacted with a (C₁-C₆) linear or branched alkyl halide, which is then optionally purified.

16. Process for the preparation of the compounds of the formula (Ib₂) according to claim 8 **characterised in that** they are obtained from the corresponding amine which is reacted with a (C₁-C₆) linear or branched alkyl halide, which is then optionally purified.

17. Pharmaceutical composition containing as active ingredient a compound according to any one of claims 1 to 9 alone or in combination with one or more inert non-toxic pharmaceutically acceptable excipients or vehicles.

18. Pharmaceutical composition according to claim 17 containing a compound according to one of claims 1 to 9 useful for the treatment of pathologies due to cartilage damage.

19. Pharmaceutical composition according to claim 17 containing a compound according to one of claims 1 or 9 useful as a diagnostic reagent capable of revealing a cartilage pathology or a metabolism.
